# EUROPEAN PATENT SPECIFICATION

(11) **EP 2 249 783 B1**
(45) Date of publication and mention of the grant of the patent: **07.04.2021**
(21) Application number: 09709387.6
(22) Date of filing: 06.02.2009
(51) Int. Cl.: A61K 8/44, A61K 8/20, A61Q 11/00, A61K 31/198, A61K 33/16, A61P 1/02

(54) **ORAL CARE PRODUCT AND METHODS OF USE THEREOF**
MUNDPFLEGEPRODUKT UND ANWENDUNGSVERFAHREN DAFÜR
PRODUIT DE SOINS BUCCO-DENTAIRES ET MÉTHODES D'UTILISATION DUDIT PRODUIT

(30) Priority: 08.02.2008 US 27431; 08.02.2008 US 27420; 08.02.2008 US 27432; 08.02.2008 US 27435; 09.02.2008 US 27441; 09.02.2008 US 27442
(43) Date of publication of application: 17.11.2010
(73) Proprietor: Colgate-Palmolive Company, New York, NY 10022 (US)
(72) Inventor: KOHLI, Rajnish, Hillsborough NJ 08844 (US); ROBINSON, Richard, Belle Mead NJ 08502 (US); SULLIVAN, Richard, Atlantic Highlands NJ 07716 (US); CUMMINS, Diane, Livingston NJ 07039 (US)
(74) Representative: Wibbelmann, Jobst
(86) International application number: PCT/US2009/033290
(87) International publication number: WO 2009/100264

(56) References cited:
- WO-A1-00/78270
- WO-A1-91/09524
- WO-A1-97/32565
- WO-A1-2004/039343
- US-A- 4 477 429
- US-A- 5 747 004
- US-A1- 2003 207 945
- US-A1- 2007 286 820
- US-B2- 6 524 558

## Description

### FIELD OF THE INVENTION

This invention relates to toothpaste compositions as defined in the claims. free or salt form and a low dosage of fluoride and to methods of using these compositions.

### BACKGROUND OF THE INVENTION

Arginine and other basic amino acids have been proposed for use in oral care and are believed to have significant benefits in combating cavity formation and tooth sensitivity. Combining these basic amino acids with minerals having oral care benefits, e.g., fluoride and calcium, to form an oral care product having acceptable long term stability, however, has proven challenging. In particular, the basic amino acid may raise the pH and facilitate dissociation of calcium ions that can react with fluoride ions to form an insoluble precipitate. Moreover, the higher pH has the potential to cause irritation. At neutral pH or acidic pH, however, a system utilizing arginine bicarbonate (which the art teaches is preferred) may release carbon dioxide, leading to bloating and bursting of the containers. Moreover, it might be expected that lowering the pH to neutral or acidic conditions would reduce the efficacy of the formulation because the arginine may form an insoluble arginine-calcium complex that has a poorer affinity for the tooth surface, and moreover that lowering the pH would reduce any effect the formulation might have on buffering cariogenic lactic acid in the mouth. Partly because of these unaddressed formulation hurdles and partly because arginine has generally been viewed in the art as a potential alternative to fluoride rather than a co-active, there has been little motivation to make oral care products comprising both arginine and fluoride. Additional hurdles are potentially posed by addition of an antimicrobial agent. Commercially available arginine-based toothpaste, such as ProClude® and DenClude®, for example, contain arginine bicarbonate and calcium carbonate, but not fluoride nor any antimicrobial agent.

At the same time, the value of antimicrobial agents, such as triclosan, in toothpaste has been recognized by many dentists. These agents however are challenging to deliver in effective amounts to the teeth and gums, and their solubility, delivery and retention on the teeth is formulation dependent. For example, triclosan (5-chloro-2-(2,4-dichlorophenoxy)phenol) is only slightly soluble in water.

Fluoride has well established benefits in oral care, particularly through the formation of fluorapatite in the teeth, which is more resistant to dissolution by acids (demineralization). However, young children can be harmed by over-exposure to fluoride, for example by swallowing large amounts of fluoridated toothpaste, leading to dental fluorosis in developing teeth, and while fluoride as used in toothpaste has an exceptional safety record, some people would nevertheless prefer to that the amount of fluoride be the absolute minimum for effectiveness.

Accordingly, there is a need for a stable oral care product that provides a basic amino acid and beneficial fluoride at a low dosage while providing effective fluoride activity.

WO-A-97/32565 discloses oral compositions containing anti-caries agents distributed in an oral vehicle. US 5,747,004 discloses a dentifrice that is capable of generating heat upon use in the oral cavity. US 2003/0207945 discloses an antimicrobial compound, compositions containing the same, and a method of using the same for reducing the presence of microorganism on a substrate or in a fluid environment. WO-A-91/09524 discloses the treatment of an impaired human immune response or to reduction of the severity of degradation of the human immune response by the administration of arginine or ornithine, or a functional analogy of arginine or ornithine, or mixtures thereof, to humans suffering from an impaired immune response or at risk of suffering an impaired immune response. US 4,477,429 discloses oral hygiene formulations incorporating N^{α}-alkyl derivatives of arginine, or the pharmaceutically acceptable salts thereof, optionally in combination with fluoride compound for combatting microorganisms, inhibiting acid production and reducing dental caries. US 6,524,558 discloses compositions and methods for calcifying dental tissue, e.g., preventing or treating dental hypersensitivity. US 2007/0286820 discloses methods of cleaning an oral surface maintaining oral health and/or increasing oral health. WO-A-00/78270 discloses compositions and methods for calcifying dental tissue, e.g. preventing or treating dental hypersensitivity. WO-A-2004/039343 discloses an oral preparation or a chewing gum comprising an organic acid and/or inorganic acid and a fluoride ion supplying compound, wherein a light scattering layer is formed inside enamel of the teeth when the oral preparation or the chewing gum is applied to teeth.

### BRIEF SUMMARY OF THE INVENTION

It is now surprisingly discovered that a basic amino acid such as arginine in combination with an amount of fluoride which would otherwise be subtherapeutic can nevertheless deliver enhanced oral care benefits, and so may promote oral health and/or systemic health, including cardiovascular health, e.g., by reducing potential for systemic infection via the oral tissues.

Without intending to be bound by a particular theory, it is hypothesized that a significant factor in the beneficial effect of arginine is that arginine and other basic amino acids can be metabolized by certain types of bacteria, e.g., *S. sanguis* which are not cariogenic and which compete with cariogenic bacteria such as *S. mutans,* for position on the teeth and in the oral cavity. The arginolytic bacteria can use arginine and other basic amino acids to produce ammonia, thereby raising the pH of their environment, while cariogenic bacteria metabolize sugar to produce lactic acid, which tends to lower the plaque pH and demineralize the teeth, ultimately leading to cavities. It is believed that regular use of a Composition of the Invention over time, will lead to a relative increase in the arginolytic bacteria and a relative decrease in the cariogenic bacteria, resulting in a higher plaque pH, in effect immunizing the teeth against cariogenic bacteria and their detrimental effects. It is believed that this pH-raising effect may be mechanistically separate from and complementary to the effect of fluoride in promoting remineralization and strengthening the tooth enamel.

Irrespective of the precise mechanism, however, it is surprisingly found that the combination of fluoride and a basic amino acid, e.g., arginine, in an oral care product according to the present invention produces unexpected benefits beyond and qualitatively different from what can be observed using compositions comprising effective amounts of either compound separately, in promoting remineralization, repairing pre-carious lesions, and enhancing oral health. Moreover, beneficial effects are still seen with levels of fluoride which would not be therapeutically effective in the absence of a basic amino acid.

It has moreover been found that this action can be further enhanced by addition of a small particle abrasive, which may act to help fill microfissures in the enamel and microtubules in the dentin. The presence of a basic amino acid in combination with an anionic surfactant is also surprisingly found to reduce bacterial adhesion to the tooth surface. Finally, the basic amino acid together with an anionic surfactant substantially enhances solubilization, release, delivery, deposition, and effectiveness of poorly soluble active agents, for example antimicrobial agents, such as triclosan.

The disclosure further encompasses methods to (i) reduce or inhibit formation of dental caries, (ii) reduce, repair or inhibit early enamel lesions, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of arginolytic bacteria, (ix) inhibit microbial biofilm formation in the oral cavity, (x) raise and/or maintain plaque pH at levels of at least pH 5.5 following sugar challenge, (xi) reduce plaque accumulation, (xii) treat dry mouth; (xiii) enhance systemic health, including cardiovascular health, e.g., by reducing potential for systemic infection via the oral tissues, (xiv) reduce erosion of the teeth, (xv) immunize the teeth against cariogenic bacteria, and/or (xvi) clean the teeth and oral cavity, comprising applying a Composition of the Invention to the oral cavity, e.g., by applying a Composition of the Invention to the oral cavity of a patient in need thereof.

### DETAILED DESCRIPTION OF THE INVENTION

The present invention provides an oral care composition according to claim 1. The disclosure provides a dentifrice (Composition 1.0) comprising,
i. an effective amount of a basic amino acid, e.g., arginine, in free or salt form; a fluoride source, e.g., a soluble fluoride salt, e.g., less than 0.1% fluoride salt, e.g., providing less than about 1000 ppm fluoride, e.g., less than about 700 ppm, e.g., from about 200 to about 600 ppm fluoride.

Further disclosed is,
1.0.1. Composition 1.0 wherein the basic amino acid is arginine, lysine, citrullene, ornithine, creatine, histidine, diaminobutanoic acid, diaminoproprionic acid, salts thereof and/or combinations thereof.
1.0.2. Composition 1.0 or 1.0.1 wherein the basic amino acid has the L-configuration.
1.0.3. Any of the preceding compositions is provided in the form of a salt of a di- or tripeptide comprising the basic amino acid.
1.0.4. Any of the preceding compositions wherein the basic amino acid is arginine.
1.0.5. Any of the preceding compositions wherein the basic amino acid is L-arginine.
1.0.6. Any of the preceding compositions wherein the basic amino acid is partially or wholly in salt form.
1.0.7. Composition 1.0.6 wherein the basic amino acid is arginine phosphate.
1.0.8. Composition 1.0.6 wherein the basic amino acid is in the form of arginine hydrochloride.
1.0.9. Composition 1.0.6 wherein the basic amino acid is arginine sulfate.
1.0.10. Composition 1.0.6 wherein the basic amino acid is arginine bicarbonate.
1.0.11. Any of the preceding compositions wherein a salt of the basic amino acid is formed in situ in the formulation by neutralization of the basic amino acid with an acid or a salt of an acid.
1.0.12. Any of the preceding compositions wherein the salt of the basic amino acid is formed by neutralization of the basic amino acid to form a premix prior to combination with the fluoride salt.
1.0.13. Any of the preceding compositions wherein the basic amino acid is present in an amount corresponding to about 0.1 to about 20%, e.g., about 1 wt. % to about 10 wt. % of the total composition weight, the weight of the basic amino acid being calculated as free base form.
1.0.14. Composition 1.0.11 wherein the basic amino acid is present in an amount of about 7.5 wt. % of the total composition weight.
1.0.15. Composition 1.0.11 wherein the basic amino acid is present in an amount of about 5 wt. % of the total composition weight.
1.0.16. Composition 1.0.11 wherein the basic amino acid is present in an amount of about 3.75 wt. % of the total composition weight.
1.0.17. Composition 1.0.11 wherein the basic amino acid is present in an amount of about 1.5 wt. % of the total composition weight.
1.0.18. Any of the preceding compositions wherein the fluoride salt is selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'-tris(2-ethanol)-dihydrofluoride), ammonium fluoride, titanium fluoride, hexafluorosulfate, and combinations thereof.
1.0.19. Any of the preceding compositions wherein the fluoride salt is a fluorophosphate.
1.0.20. Any of the preceding composition wherein the fluoride salt is sodium monofluorophosphate.
1.0.21. Any of the preceding compositions where the fluoride salt is sodium fluoride.
1.0.22. Any of the preceding compositions wherein the fluoride salt is present in an amount of about 0.01 wt. % to about 0.1 wt. % of the total composition weight.
1.0.23. Any of the preceding compositions wherein the fluoride salt provides fluoride ion in an amount of about 0.05 to about 0.1 wt. % of the total composition weight.
1.0.24. Any of the preceding compositions wherein the soluble fluoride salt provides fluoride ion in an amount of from about 50 to about 600 ppm.
1.0.25. Any of the preceding compositions which is a mouthwash having about 50 to about 250 ppm available fluoride ion.
1.0.26. Any of the preceding compositions which is a dentifrice having about 100 to about 500 ppm available fluoride ion.
1.0.27. Any of the preceding compositions wherein the pH is about 6 to about 9, e.g., about 6.5 to about 7.4 or about 7.5 to about 9.
1.0.28. Any of the preceding compositions wherein the pH is about 6.5 to about 7.4.
1.0.29. Any of the preceding compositions wherein the pH is about 6.8 to about 7.2.
1.0.30. Any of the preceding compositions wherein the pH is approximately neutral.
1.0.31. Any of the preceding compositions further comprising an abrasive or particulate.
1.0.32. The immediately preceding composition wherein the adhesive or particulate is selected from sodium bicarbonate, calcium phosphate (e.g.,dicalcium phosphate dihydrate), calcium sulfate, precipitated calcium carbonate, silica (e.g., hydrated silica), iron oxide, aluminum oxide, perlite, plastic particles, e.g., polyethylene, and combinations thereof.
1.0.33. The immediately preceding composition wherein the abrasive or particulate is selected from a calcium phosphate (e.g., dicalcium phosphate dihydrate), calcium sulfate, precipitated calcium carbonate, silica (e.g., hydrated silica), and combinations thereof.
1.0.34. Any of the preceding compositions comprising an abrasive in an amount of about 15 wt. % to about 70 wt. % of the total composition weight.
1.0.35. Any of the preceding compositions comprising a small particle abrasive fraction of at least about 5% having a d50 of less than about 5 micrometers.
1.0.36. Any of the preceding compositions having an RDA of less than about 150, e.g., about 40 to about 140.
1.0.37. Any of the preceding compositions wherein the anionic surfactant is selected from
   a. water-soluble salts of higher fatty acid monoglyceride monosulfates (e.g., the sodium salt of the monosulfated monoglyceride of hydrogenated coconut oil fatty acids such as sodium N-methyl N-cocoyl taurate, sodium cocomo-glyceride sulfate),
   b. higher alkyl sulfates, e.g., sodium lauryl sulfate,
   c. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K (for example sodium laureth-2 sulfate (CH₃(CH₂)₁₀CH₂(OCH₂CH₂)₂OSO₃Na)),
   d. higher alkyl aryl sulfonates (such as sodium dodecyl benzene sulfonate (sodium lauryl benzene sulfonate)),
   e. higher alkyl sulfoacetates (such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate),
   f. and mixtures thereof.

By "higher alkyl" is meant, e.g., C₆₋₃₀ alkyl. In particular embodiments, the anionic surfactant is selected from sodium lauryl sulfate and sodium ether lauryl sulfate.
1.0.38. Any of the preceding compositions wherein the anionic surfactant is selected from sodium lauryl sulfate, sodium ether lauryl sulfate, and mixtures thereof.
1.0.39. Any of the preceding compositions wherein the anionic surfactant is present in an amount of from about 0.3% to about 4.5% by weight.
1.0.40. Any of the preceding compositions additionally comprising surfactants selected from cationic, zwitterionic, and nonionic surfactants, and mixtures thereof.
1.0.41. Any of the preceding compositions further comprising at least one humectant.
1.0.42. Any of the preceding compositions further comprising at least one humectant selected from glycerin, sorbitol, xylitol, and combinations thereof.
1.0.43. Any of the preceding compositions further comprising xylitol, solbrol, or chitosan.
1.0.44. Any of the preceding compositions further comprising at least one polymer.
1.0.45. Any of the preceding compositions further comprising at least one polymer selected from polyethylene glycols, polyvinylmethyl ether maleic acid copolymers, polysaccharides (e.g., cellulose derivatives, for example carboxymethyl cellulose, or polysaccharide gums, for example xanthan gum or carrageenan gum), and combinations thereof.
1.0.46. Any of the preceding compositions further comprising gum strips or fragments.
1.0.47. Any of the preceding compositions further comprising flavoring, fragrance and/or coloring.
1.0.48. Any of the preceding compositions further comprising water.
1.0.49. Any of the preceding compositions further comprising an antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.0.50. Any of the preceding compositions further comprising an anti-inflammatory compound, e.g., an inhibitor of at least one of host pro-inflammatory factors selected from matrix metalloproteinases (MMPs), cyclooxygenases (COX), PGE₂, interleukin 1 (IL-1), IL-1β converting enzyme (ICE), transforming growth factor β1 (TGF-β1), inducible nitric oxide synthase (iNOS), hyaluronidase, cathepsins, nuclear factor kappa B (NF-κB), and IL-1 Receptor Associated Kinase (IRAK), e,g, selected from aspirin, ketorolac, flurbiprofen, ibuprofen, naproxen, indomethacin, aspirin, ketoprofen, piroxicam, meclofenamic acid, nordihydoguaiaretic acid, and mixtures thereof.
1.0.51. Any of the preceding compositions comprising an antioxidant, e.g., selected from the group consisting of Co-enzyme Q10, PQQ, Vitamin C, Vitamin E, Vitamin A, anethole-dithiothione, and mixtures thereof.
1.0.52. Any of the preceding compositions wherein the anti-microbial is poorly soluble.
1.0.53. Any of the preceding compositions further comprising triclosan.
1.0.54. Any of the preceding composition comprising triclosan and Zn²⁺ ion source, e.g., zinc citrate.
1.0.55. Any of the preceding compositions further comprising triclosan and xylitol.
1.0.56. Any of the preceding compositions further comprising triclosan, xylitol, and precipitated calcium carbonate.
1.0.57. Any of the preceding compositions further comprising an anti-calculus agent.
1.0.58. Any of the preceding compositions further comprising an anti-calculus agent which is a polyphosphate, e.g., pyrophosphate, tripolyphosphate, or hexametaphosphate, e.g., in sodium salt form.
1.0.59. Any of the preceding compositions further comprising an antibacterial agent in an amount of about 0.01 to about 5 wt. % of the total composition weight.
1.0.60. Any of the preceding compositions further comprising triclosan in an amount of about 0.01 to about 1 wt. percent of the total composition weight.
1.0.61. Any of the preceding compositions further comprising triclosan in an amount of about 0.3% of the total composition weight.
1.0.62. Any of the preceding compositions further comprising a whitening agent.
1.0.63. Any of the preceding compositions further comprising a whitening agent selected from a whitening active selected from the group consisting of peroxides, metal chlorites, perborates, percarbonates, peroxyacids, hypochlorites, and combinations thereof
1.0.64. Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate), or hydrogen peroxide polymer complexes such as hydrogen peroxide-polyvinyl pyrrolidone polymer complexes.
1.0.65. Any of the preceding compositions further comprising an agent that interferes with or prevents bacterial attachment, e.g., solbrol or chitosan.
1.0.66. Any of the preceding compositions further comprising a source of calcium and phosphate selected from (i) calcium-glass complexes, e.g., calcium sodium phosphosilicates, and (ii) calcium-protein complexes, e.g., casein phosphopeptide-amorphous calcium phosphate.
1.0.67. Any of the preceding compositions further comprising a soluble calcium salt, e.g., selected from calcium sulfate, calcium chloride, calcium nitrate, calcium acetate, calcium lactate, and combinations thereof.
1.0.68. Any of the preceding compositions further comprising a physiologically acceptable potassium salt, e.g., potassium nitrate or potassium chloride, in an amount effective to reduce dentinal sensitivity.
1.0.69. Any of the preceding compositions comprising from about 0.1% to about 7.5% of a physiologically acceptable potassium salt, e.g., potassium nitrate and/or potassium chloride.
1.0.70. Any of the preceding compositions which is a toothpaste comprising an arginine salt, e.g., arginine hydrochloride, arginine phosphate or arginine bicarbonate; triclosan; an anionic surfactant, e.g., sodium lauryl sulfate; and a soluble fluoride salt, e.g., sodium monofluorophosphate or sodium fluoride.
1.0.71. Any of the preceding compositions effective upon application to the oral cavity, e.g., with brushing, to (i) reduce or inhibit formation of dental caries, (ii) reduce, repair or inhibit early enamel lesions, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of arginolytic bacteria, (ix) inhibit microbial biofilm formation in the oral cavity, (x) raise and/or maintain plaque pH at levels of at least pH about 5.5 following sugar challenge, (xi) reduce plaque accumulation, (xii) treat, relieve or reduce dry mouth, (xiii) enhance systemic health, including cardiovascular health, e.g., by reducing potential for systemic infection via the oral tissues, (xiv) whiten teeth, (xv) reduce erosion of the teeth, (xvi) immunize the teeth against cariogenic bacteria, and/or (xvii) clean the teeth and oral cavity.
1.0.72. A composition obtained or obtainable by combining the ingredients as set forth in any of the preceding compositions.
1.0.73. Any of the preceding compositions in a form selected from mouthrinse, toothpaste, tooth gel, tooth powder, non-abrasive gel, mousse, foam, mouth spray, lozenge, oral tablet, dental implement, and pet care product.
1.0.74. Any of the preceding compositions wherein the composition is toothpaste.
1.0.75. Any of the preceding compositions wherein the composition is a toothpaste optionally further comprising one or more of one or more of water, abrasives, surfactants, foaming agents, vitamins, polymers, enzymes, humectants, thickeners, antimicrobial agents, preservatives, flavorings, colorings and/or combinations thereof.
1.0.76. Any of the preceding compositions 1.0 - 1.0.72 wherein the composition is a mouthwash.
1.0.77. Any of the preceding compositions further comprising a breath freshener, fragrance or flavoring.

The disclosure encompasses a Composition (Composition 1.1) e.g., according to any of the preceding Compositions 1.0 - 1.0.77, comprising
i. an effective amount of a salt of a basic amino acid;
ii. an effective amount of a soluble fluoride salt;
iii. an anionic surfactant, e.g., sodium lauryl sulfate;
iv. an anionic polymer, e.g., a copolymer of methyl vinyl ether and maleic anhydride; and
v. an antibacterial agent, e.g., triclosan.

The disclosure encompasses a Composition (Composition 1.2) e.g., according to any of the preceding Compositions 1.0 - 1.0.77, comprising
i. an effective amount of a salt of a basic amino acid;
ii. an antibacterial agent, e.g., triclosan;
iii. an effective amount of a soluble fluoride salt; and
iv. small particle abrasive, such that the composition has an RDA of less than about 160, e.g., about 40 to about 140, e.g., comprising at least about 5% , e.g., at least about 20% of an abrasive having a d50 less than about 5 micrometers, e.g., silica having a d50 of about 3 to about 4 micrometers.

The disclosure encompasses a method (Method 2) to improve oral health comprising applying an effective amount of the oral composition of any of the embodiments under Compositions 1.0, 1.1, or 1.2, to the oral cavity of a subject e.g., a method to
i. reduce or inhibit formation of dental caries,
ii. reduce, repair or inhibit early enamel lesions, e.g., as detected by quantitative light-induced fluorescence (QLF) or electrical caries measurement (ECM),
iii. reduce or inhibit demineralization and promote remineralization of the teeth,
iv. reduce hypersensitivity of the teeth,
v. reduce or inhibit gingivitis,
vi. promote healing of sores or cuts in the mouth,
vii. reduce levels of acid producing bacteria,
viii. to increase relative levels of arginolytic bacteria,
ix. inhibit microbial biofilm formation in the oral cavity,
x. raise and/or maintain plaque pH at levels of at least about pH 5.5 following sugar challenge,
xi. reduce plaque accumulation,
xii. treat dry mouth,
xiii. enhance systemic health, including cardiovascular health, e.g., by reducing potential for systemic infection via the oral tissues,
xiv. whiten teeth,
xv. immunize teeth against cariogenic bacteria,
xvi. to reduce erosion of the teeth, and/or
xvii. clean the teeth and oral cavity.

The disclosure further comprises the use of arginine in the manufacture of a Composition of the Invention, e.g., for use in any of the indications set forth in Method 2.

### Basic Amino Acids

According to the invention, the basic amino acid is arginine, for example, L-arginine, or a salt thereof.

The compositions of the invention are intended for topical use in the mouth and so salts for use in the present invention should be safe for such use, in the amounts and concentrations provided. Suitable salts include salts known in the art to be pharmaceutically acceptable salts are generally considered to be physiologically acceptable in the amounts and concentrations provided. Physiologically acceptable salts include those derived from pharmaceutically acceptable inorganic or organic acids or bases, for example acid addition salts formed by acids which form a physiological acceptable anion, e.g., hydrochloride or bromide salt, and base addition salts formed by bases which form a physiologically acceptable cation, for example those derived from alkali metals such as potassium and sodium or alkaline earth metals such as calcium and magnesium. Physiologically acceptable salts may be obtained using standard procedures known in the art, for example, by reacting a sufficiently basic compound such as an amine with a suitable acid affording a physiologically acceptable anion.

In various embodiments, the basic amino acid is present in an amount of about 0.5 wt. % to about 20 wt. % of the total composition weight, about 1 wt. % to about 10 wt. % of the total composition weight, for example about 1.5 wt. %, about 3.75 wt. %, about 5 wt. %, or about 7.5 wt. % of the total composition weight.

RDA: RDA is an abbreviation for radioactive dentin abrasion, a relative measure of abrasivity. Typically, extracted human or cow teeth are irradiated in a neutron flux, mounted in methylmethacrylate (bone glue), stripped of enamel, inserted into a brushing-machine, brushed by American Dental Association (ADA) standards (reference toothbrush, 150g pressure, 1500 strokes, 4-to-1 water-toothpaste slurry). The radioactivity of the rinse water is then measured and recorded. For experimental control, the test is repeated with an ADA reference toothpaste made of calcium pyrophosphate, with this measurement given a value of 100 to calibrate the relative scale.

Fluoride Ion Source: The oral care compositions include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al..

Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof.

The oral care compositions of the invention contain a source of fluoride ions in amounts sufficient to supply 300 to 600 ppm.

Weights of fluoride salts to provide the appropriate level of fluoride ion will obviously vary based on the weight of the counter ion in the salt.

### Abrasives

The Compositions of the Invention may comprise a calcium phosphate abrasive, e.g., tricalcium phosphate (Ca₃(PO₄)₂), hydroxyapatite (Ca₁₀(PO₄)₆(OH)₂), dicalcium phosphate dihydrate (CaHPO₄ • 2H₂O, also sometimes referred to herein as DiCal), or calcium pyrophosphate.

The compositions may include one or more additional abrasives, for example silica abrasives such as precipitated silicas having a mean particle size of up to about 20 microns, such as Zeodent 115®, marketed by J. M. Huber. Other useful abrasives also include sodium metaphosphate, potassium metaphosphate, aluminum silicate, calcined alumina, bentonite or other siliceous materials, or combinations thereof.

The silica abrasive polishing materials useful herein, as well as the other abrasives, generally have an average particle size ranging between about 0.1 and about 30 microns, about 5 to about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason.

In certain embodiments, abrasive materials useful in the practice of the oral care compositions in accordance with the invention include silica gels and precipitated amorphous silica having an oil absorption value of about less than about 100 cc/100 g silica and in the range of about 45 cc/100 g to about 70 cc/100 g silica. Oil absorption values are measured using the ASTM Rub-Out Method D281. In certain embodiments, the silicas are colloidal particles having an average particle size of about 3 microns to about 12 microns, and about 5 to about 10 microns.

In particular embodiments, the abrasive materials comprise a large fraction of very small particles, e.g., having a d50 less than about 5 microns, for example, small particle silica (SPS) having a d50 of about 3 to about 4 microns, for example Sorbosil AC43® (Ineos). Such small particles are particularly useful in formulations targeted at reducing hypersensitivity. The small particle component may be present in combination with a second larger particle abrasive. In certain embodiments, for example, the formulation comprises about 3 to about 8% SPS and about 25 to about 45% of a conventional abrasive.

Low oil absorption silica abrasives particularly useful in the practice of the invention are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of about 29% by weight averaging about 7 to about 10 microns in diameter, and an oil absorption of less than about 70 cc/100 g of silica is an example of a low oil absorption silica abrasive useful in the practice of the present invention. The abrasive is present in the oral care composition of the present invention at a concentration of about 10 to about 60% by weight, in other embodiment about 20 to about 45% by weight, and in another embodiment about 30 to about 50% by weight.

### Agents to Increase the Amount of Foaming

The oral care compositions of the invention also may include an agent to increase the amount of foam that is produced when the oral cavity is brushed.

Illustrative examples of agents that increase the amount of foam include, but are not limited to polyoxyethylene and certain polymers including, but not limited to, alginate polymers.

The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox® is the trade name for the high molecular weight polyoxyethylene produced by Union Carbide.

The polyoxyethylene may be present in an amount of about 1% to about 90%, in one embodiment about 5% to about 50% and in another embodiment about 10% to about 20% by weight of the oral care carrier component of the oral care compositions of the present invention. The dosage of foaming agent in the oral care composition (i.e., a single dose) is about 0.01 to about 0.9 % by weight, about 0.05 to about 0.5% by weight, and in another embodiment about 0.1 to about 0.2 % by weight.

### Surfactants

The Compositions of the Invention contain an anionic surfactant as defined in the claims.
Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al..

In another embodiment, cationic surfactants useful in the present invention can be broadly defined as derivatives of aliphatic quaternary ammonium compounds having one long alkyl chain containing about 8 to about 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof.

Illustrative cationic surfactants are the quaternary ammonium fluorides described in U.S. Pat. No. 3,535,421, to Briner et al.. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In certain embodiments, zwitterionic synthetic surfactants useful in the present invention can be broadly described as derivatives of aliphatic quaternary ammonium, phosphomium, and sulfonium compounds, in which the aliphatic radicals can be straight chain or branched, and wherein one of the aliphatic substituents contains about 8 to about 18 carbon atoms and one contains an anionic water-solubilizing group, e.g., carboxy, sulfonate, sulfate, phosphate or phosphonate. Illustrative examples of the surfactants suited for inclusion into the composition include, but are not limited to, sodium alkyl sulfate, sodium lauroyl sarcosinate, cocoamidopropyl betaine and polysorbate 20, and combinations thereof.

In a particular embodiment, the Composition of the Invention comprises sodium lauryl sulfate.

The surfactant or mixtures of compatible surfactants can be present in the compositions of the present invention in about 0.1% to about 5%, in another embodiment about 0.3% to about 3% and in another embodiment about 0.5% to about 2% by weight of the total composition.

### Flavoring Agents

The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of about 0.1 to about 5% by weight and about 0.5 to about 1.5% by weight. The dosage of flavoring agent in the individual oral care composition dosage (i.e., a single dose) is about 0.001 to about 0.05% by weight and in another embodiment about 0.005 to 0.015 % by weight.

### Chelating agents

The oral care compositions of the invention also may optionally include one or more chelating agents able to complex calcium found in the cell walls of the bacteria. Binding of this calcium weakens the bacterial cell wall and augments bacterial lysis.

Another group of agents suitable for use as chelating agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least about 1 wt. % pyrophosphate ions, about 1.5 wt. % to about 6 wt. %, about 3.5 wt. % to about 6 wt. % of such ions.

### Polymers

The oral care compositions of the invention also optionally include one or more polymers, such as polyethylene glycols, polyvinylmethyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Particularly when noncationic antibacterial agents or antibacterial agents, e.g., triclosan, are included in any of the dentifrice components, there is also preferably included from about 0.05 to about 5% of an agent which enhances the delivery and retention of the agents to, and retention thereof on oral surfaces. Such agents useful in the present invention are disclosed in U.S. Pat. Nos. 5,188,821 and 5,192,531; and include synthetic anionic polymeric polycarboxylates, such as about 1:4 to about 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 30,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

Another useful class of polymeric agents includes polyamino acids, particularly those containing proportions of anionic surface-active amino acids such as aspartic acid, glutamic acid and phosphoserine, as disclosed in U.S. Pat. No. 4,866,161 Sikes et al..

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5% by weight of the total composition are used.

### Enzymes

The oral care compositions of the invention may also optionally include one or more enzymes. Useful enzymes include any of the available proteases, glucanohydrolases, endoglycosidases, amylases, mutanases, lipases and mucinases or compatible mixtures thereof. In certain embodiments, the enzyme is a protease, dextranase, endoglycosidase and mutanase. In another embodiment, the enzyme is papain, endoglycosidase or a mixture of dextranase and mutanase. Additional enzymes suitable for use in the present invention are disclosed in U.S. Pat. No. 5,000,939 to Dring et al., U.S. Pat. No. 4,992,420; U.S. Pat. No. 4,355,022; U.S. Pat. No. 4,154,815; U.S. Pat. No. 4,058,595; U.S. Pat. No. 3,991,177; and U.S. Pat. No. 3,696,191. An enzyme of a mixture of several compatible enzymes in the current invention constitutes about 0.002% to about 2% in one embodiment or about 0.05% to about 1.5% in another embodiment or in yet another embodiment about 0.1% to about 0.5%.

### Water

Water may also be present in the oral compositions of the invention. Water, employed in the preparation of commercial oral compositions should be deionized and free of organic impurities. Water commonly makes up the balance of the compositions and includes about 10% to about 90%, about 20% to about 60% or about 10% to about 30% by weight of the oral compositions. This amount of water includes the free water which is added plus that amount which is introduced with other materials such as with sorbitol or any components of the invention.

### Humectants

Within certain embodiments of the oral compositions, it is also desirable to incorporate a humectant to prevent the composition from hardening upon exposure to air. Certain humectants can also impart desirable sweetness or flavor to dentifrice compositions. The humectant, on a pure humectant basis, generally includes about 15% to about 70% in one embodiment or about 30% to about 65% in another embodiment by weight of the dentifrice composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the toothpaste compositions herein.

In addition to the above described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele.

### Methods of Manufacture

The compositions of the present invention can be made using methods which are common in the oral product area.

In one illustrative embodiment, the oral care composition is made by neutralizing or partially neutralizing arginine in a gel phase with an acid, e.g., phosphoric acid, hydrochloric acid or carbonic acid, and mixing to form Premix 1.

Actives such as, for example, vitamins, CPC, fluoride, abrasives, and any other desired active ingredients are added to Premix 1 and mixed to form Premix 2.

A toothpaste base, for example, dicalcium phosphate or silica, is added to Premix 2 and mixed. The final slurry is formed into an oral care product.

### Composition Use

The compositions according to the invention are useful to a method to protect the teeth by facilitating repair and remineralization, in particular to reduce or inhibit formation of dental caries, reduce or inhibit demineralization and promote remineralization of the teeth, reduce hypersensitivity of the teeth, and reduce, repair or inhibit early enamel lesions, e.g., as detected by quantitative light-induced fluorescence (QLF) or electronic caries monitor (ECM).

Quantitative Light-induced Fluorescence is a visible light fluorescence that can detect early lesions and longitudinally monitor the progression or regression. Normal teeth fluoresce in visible light; demineralized teeth do not or do so only to a lesser degree. The area of demineralization can be quantified and its progress monitored. Blue laser light is used to make the teeth auto fluoresce. Areas that have lost mineral have lower fluorescence and appear darker in comparison to a sound tooth surface. Software is used to quantify the fluorescence from a white spot or the area/volume associated with the lesion. Generally, subjects with existing white spot lesions are recruited as panelists. The measurements are performed in vivo with real teeth. The lesion area/volume is measured at the beginning of the clinical. The reduction (improvement) in lesion area/volume is measured at the end of 6 months of product use. The data is often reported as a percent improvement versus baseline.

Electrical Caries Monitoring is a technique used to measure mineral content of the tooth based on electrical resistance. Electrical conductance measurement exploits the fact that the fluid-filled tubules exposed upon demineralization and erosion of the enamel conduct electricity. As a tooth loses mineral, it becomes less resistive to electrical current due to increased porosity. An increase in the conductance of the patient's teeth therefore may indicate demineralization. Generally, studies are conducted of root surfaces with an existing lesion. The measurements are performed in vivo with real teeth. Changes in electrical resistance before and after 6 month treatments are made. In addition, a classical caries score for root surfaces is made using a tactile probe. The hardness is classified on a three point scale: hard, leathery, or soft. In this type of study, typically the results are reported as electrical resistance (higher number is better) for the ECM measurements and an improvement in hardness of the lesion based on the tactile probe score.

The Compositions of the Invention are thus useful in a method to reduce early lesions of the enamel (as measured by QLF or ECM) relative to a composition lacking effective amounts of fluorine and/or arginine.

The Compositions of the invention are additionally useful in methods to reduce harmful bacteria in the oral cavity, for example methods to reduce or inhibit gingivitis, reduce levels of acid producing bacteria, to increase relative levels of arginolytic bacteria, inhibit microbial biofilm formation in the oral cavity, raise and/or maintain plaque pH at levels of at least pH about 5.5 following sugar challenge, reduce plaque accumulation, and/or clean the teeth and oral cavity.

Finally, by increasing the pH in the mouth and discouraging pathogenic bacteria, the Compositions of the Invention are useful to promote healing of sores or cuts in the mouth.

The compositions according to the invention can be incorporated into oral compositions for the care of the mouth and teeth such as toothpastes, transparent pastes, gels, mouth rinses, sprays and chewing gum.

Levels of antibacterial will vary similarly, with levels used in toothpaste being e.g., about 5 to about 15 times greater than used in mouthrinse. For example, a triclosan mouthrinse may contain, e.g., about 0.03 wt % triclosan while a triclosan toothpaste may contain about 0.3 wt % triclosan.

Enhancing oral health also provides benefits in systemic health, as the oral tissues can be gateways for systemic infections. Good oral health is associated with systemic health, including cardiovascular health. The compositions of the invention provide particular benefits because basic amino acids, especially arginine, are sources of nitrogen which supply NO synthesis pathways and thus enhance microcirculation in the oral tissues. Providing a less acidic oral environment is also helpful in reducing gastric distress and creates an environment less favorable to Heliobacter, which is associated with gastric ulcers. Arginine in particular is required for high expression of specific immune cell receptors, for example T-cell receptors, so that arginine can enhance an effective immune response. The compositions of the invention are thus useful to enhance systemic health, including cardiovascular health.

As used throughout, ranges are used as shorthand for describing each and every value that is within the range. Any value within the range can be selected as the terminus of the range. In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible.

### EXAMPLES

### Example 1 - Availability and Delivery of Fluoride in Arginine Formulations (not claimed)

Formulations are prepared using commercial toothpaste comprising, i.a., 0.3% by weight of triclosan, 0.075% by weight of sodium fluoride, sodium lauryl sulfate, and a copolymer of methyl vinyl ether and maleic anhydride (PVM/MA), to which is added 0, 1%, 3%, and 5% L-arginine hydrochloride (pH 7.0).

Incorporation of L-arginine in the commercial formulation enhances the amount of soluble fluoride available from the formulation. This enhanced delivery leads directly to an enhancement of delivery of fluoride effect of the formulation.

## Claims

1. A toothpaste composition comprising
a. an effective amount of arginine, in free or salt form;
b. a fluoride ion source which provides from 300 to 600 ppm by weight of fluoride ions per weight of the composition;
c. and an anionic surfactant wherein the anionic surfactant is selected from sodium lauryl sulfate; sodium laureth sulfate; and mixtures thereof,
wherein arginine is present in an amount of 1 to 10 weight % of the total composition, the weight of the arginine being calculated as free base form.

## Patentansprüche

1. Zahnpastazusammensetzung, umfassend
a. eine wirksame Menge an Arginin in freier Form oder Salzform;
b. eine Fluoridionenquelle, die, bezogen auf das Gewicht, 300 bis 600 ppm an Fluoridionen bezogen auf das Gewicht der Zusammensetzung bereitstellt;
c. und ein anionisches oberflächenaktives Mittel, wobei das anionische oberflächenaktive Mittel ausgewählt ist aus Natriumlaurylsulfat; Natriumlaurethsulfat; und Mischungen davon,
wobei Arginin in einer Menge von 1 bis 10 Gewichts-% der Gesamtzusammensetzung vorliegt, wobei das Gewicht des Arginins als freie Basenform berechnet wird.

## Revendications

1. Composition de pâte dentifrice comprenant
a. une quantité efficace d'arginine, sous forme libre ou sous forme de sel ;
b. une source d'ions fluorure qui fournit de 300 à 600 ppm en poids d'ions fluorure par poids de la composition ;
c. et un tensioactif anionique dans lequel le tensioactif anionique est choisi parmi le laurylsulfate de sodium ; le laureth sulfate de sodium ; et leurs mélanges,
dans laquelle de l'arginine est présente en une quantité de 1 à 10 % en poids de la composition totale, le poids de l'arginine étant calculé sous forme de base libre.
